# EUROPEAN PATENT SPECIFICATION

(11) **EP 1 933 884 B1**
(45) Date of publication and mention of the grant of the patent: **06.09.2017**
(21) Application number: 06836284.7
(22) Date of filing: 11.10.2006
(51) Int. Cl.: A61K 49/10, A61B 5/07, A61B 5/055

(54) **IMAGING AGENTS AND METHODS OF USE THEREOF**
ABBILDUNGSWIRKSTOFFE UND VERFAHREN ZU IHRER VERWENDUNG
AGENTS D'IMAGERIE ET LEURS PROCÉDÉS D'UTILISATION

(30) Priority: 11.10.2005 US 725439 P
(43) Date of publication of application: 25.06.2008
(73) Proprietor: Huntington Medical Research Institutes, Pasadena, CA 91105 (US)
(72) Inventor: ROSS, Brian, D., Altadena, CA 91001 (US); BHATTACHARYA, Pratip, Pasadena, CA 91106 (US)
(74) Representative: Gibson, Mark
(86) International application number: PCT/US2006/039974
(87) International publication number: WO 2007/044867

(56) References cited:
- US-A- 5 492 814
- US-B1- 6 574 496
- US-B2- 6 711 440
- BHATTACHARYA P ET AL: "Ultra-fast three dimensional imaging of hyperpolarized 13C in vivo", MAGNETIC RESONANCE MATERIALS IN PHYSICS, BIOLOGY AND MEDICINE, CHAPMAN AND HALL, LONDON, GB, vol. 18, no. 5, 1 October 2005 (2005-10-01), pages 245-256, XP019358223, ISSN: 1352-8661
- E. JOHANSSON ET AL: "Perfusion assessment with bolus differentiation: A technique applicable to hyperpolarized tracers", MAGNETIC RESONANCE IN MEDICINE, vol. 52, no. 5, 1 November 2004 (2004-11-01), pages 1043-1051, XP055044107, ISSN: 0740-3194, DOI: 10.1002/mrm.20247
- GOLMAN K ET AL: "Parahydrogen-induced polarization in imaging: subsecond <13>C angiography", MAGNETIC RESONANCE IN MEDICINE, ACADEMIC PRESS, DULUTH, MN, US, vol. 46, no. 1, 1 July 2001 (2001-07-01), pages 1-5, XP002263068, ISSN: 0740-3194, DOI: 10.1002/MRM.1152
- ALEXANDER P. LIN ET AL: "Reduced glutamate neurotransmission in patients with Alzheimer's disease?an in vivo 13C magnetic resonance spectroscopy study", MAGMA MAGNETIC RESONANCE MATERIALS IN PHYSICS, BIOLOGY AND MEDICINE, vol. 16, no. 1, 1 February 2003 (2003-02-01), pages 29-42, XP055044228, ISSN: 0968-5243, DOI: 10.1007/s10334-003-0004-x

## Description

### FIELD OF INVENTION

The invention relates to compositions and methods useful in connection with magnetic resonance imaging.

### BACKGROUND OF THE INVENTION

Magnetic resonance ("MR") imaging has become a well-accepted and commonly-used technique for studying a wide range of physiologic processes. This technology is useful in connection with disease diagnosis and prognosis, and in the broader study of biological systems. Indeed, many hospitals and medical facilities have MR imaging equipment on-site, and routinely make use of it to aid in the diagnosis and monitoring of an array of diseases and physiologic conditions. Contrast agents or reporter molecules are used in connection with MR imaging, and a wide range of products is commercially available to image various systems.

Boltzmann Distribution leads to low signal to noise ratio ("SNR") in nuclear MR spectroscopy. This has not limited the application of nuclear MR spectroscopy in chemistry where time and conditions to acquire a spectrum is less of an issue. However, in biology and particularly in medicine, nuclear MR spectroscopy has yet to reach its full potential, at least in part because of the time limitation associated with the high number of transients required to obtain a well-resolved spectrum of the poorly magnetized biological material at room temperature. Furthermore, MR imaging and MR spectroscopy remain expensive (only about 15-20 patients per day, per instrument) and are limited by patient tolerance. The high cost of this technique has also limited its usage mostly to the developed nations; thereby restricting its social and humanitarian impact.

Para-hydrogen can be used for creating highly polarized nuclei, exceeding the thermal equilibrium polarization determined by the Boltzmann Distribution by several orders of magnitude. The PASADENA (Parahydrogen and Synthesis Allows Dramatically Enhanced Nuclear Alignment) phenomenon discovered in 1986 by Bowers and Weitekamp [C.R. Bowers and D.P. Weitekamp, Transformation of symmetrization order to nuclear-spin magnetization by chemical reaction and nuclear magnetic resonance, Phys. Rev. Lett., 57(21):2645-2648 (1986); C.R. Bowers and D.P. Weitekamp, Parahydrogen and Synthesis Allow Dramatically Enhanced Nuclear Alignment, J. Am. Chem. Soc., 109:5541-5542 (1987)] creates a non-equilibrium spin order that can be transformed into polarization. The first biomedical application of the technique was reported in 2001 [K. Golman et al., Parahydrogen-induced polarization in imaging: subsecond (13)C angiography, Magn. Reson. Med., 46:1-5 (2001)]. The transfer of this spin order into polarization of a suitable hetero nucleus can be accomplished by either a diabatic field-cycling scheme [*Id.;* J.H. Ardenkjaer-Larsen et al., Increase in signal-to-noise ratio of > 10,000 times in liquid-state NMR, Proc. Natl. Acad. Sci. USA, 100(18):10158-63 (2003); H. Johannesson et al., Transfer of para-hydrogen spin order into polarization by diabatic field cycling, C. R. Physique, 5:315-324 (2004)], or by RF pulses, before administration of the contrast agent to subjects.
The PASADENA phenomenon creates a non-equilibrium spin order within seconds at liquid state temperatures that can be transformed into polarization (P) close to unity. It is well known [S. Forsen and R.A. Hoffman, Study of moderately rapid exchange reaction by means of nuclear magnetic double resonance, J. Chem. Phys., 39: 2892-2901 (1963)] that P for a given nucleus is conserved through chemical reactions, relaxing toward the equilibrium value with a characteristic time T₁ of up to several tens of seconds for ¹³C [R.R. Ernst et al., Principles of Magnetic Resonance in One and Two Dimensions, Oxford University Press (Oxford, UK; 1990)]. Thus, the establishment by any method of a high value of P allows the corresponding sensitivity enhancement to be transported to any location and chemical species that can be reached on this time scale. Recent work [K. Golman, et al., Molecular imaging using hyperpolarized 13C, British J. of Radiol., 76: S118-S127 (2003); P. Bhattacharya et al., Ultra fast Steady State Free Precession Imaging of Hyperpolarized 13C In Vivo". MAGMA, 18.5, 245-256 (2005).] has demonstrated ¹³C polarizations in excess of 20% (P>0.2) for the nascent products of molecular addition of dihydrogen and sub-second MR imaging of these products following arterial injection. Even after three times T₁, sufficient time for an injected species to be taken up from the blood and metabolized, the polarization has decayed to P = 10-². A MR spectroscopy or imaging pulse sequence initiated at this time provides a sensitivity that is over 10,000 times greater than the signal from the same population of molecules at equilibrium [J.H. Ardenkjaer-Larsen *et al.* at 10158]. This corresponds to a usable SNR ratio for a single repetition of this experiment that would otherwise require 10⁸ repetitions (at least 10⁸ s = 3 years).

The nuclear spin states of hydrogen are obtained from the eigenstates of the Hamiltonian that describes a two-spin system. Because of their vanishing chemical shift difference, the protons are strongly coupled resulting in an A2 spin system with three symmetric spin states and one anti-symmetric spin state. Due to quantum statistical mechanical properties, the symmetric states (ortho-hydrogen) are correlated with odd-valued rotational states, and the anti-symmetric (para-hydrogen) with even-valued rotational states. For such an ensemble of states the system is best described using density operator formalism. In the high temperature limit there is an equal population of the four spin states. At low enough temperatures the population of the lowest rotational state dominates, and an excess of para-hydrogen is present.

Some MR imaging technologies relate to the use of contrast agents enriched with stable-isotope ¹³C (as well as ¹⁵N and other imaging nuclei) in conjunction with PASADENA polarization. By way of example, U.S. Patent No. 6,574,495 and patents related thereto describe various aspects of this technology. However, the full potential of PASADENA polarization has not heretofore been realized in MR imaging. Only a small handful of reporter molecules have been fully described in the art, and the reactive properties of these molecules *in vivo* have not been exploited.

In one example of a field in which MR imaging may find application, among the growing health problems in our society is sudden death due to coronary artery occlusion and myocardial infarction. While this was predominantly a male disease of the 4^{th} and 5^{th} decades, it is increasingly seen in women and is often "silent;" that is, current non-invasive and invasive cardiac investigations fail to identify the problem before catastrophic coronary occlusion. Increasing evidence of inflammatory disease underlying the classical events of atherosclerosis has lead to the concept of "vulnerable plaque." Calcification, long believed to predict coronary artery occlusion, and the basis of Electron Beam Tomography (EBT) as a health "screen," is no longer considered the important element in coronary occlusion. Instead, the rapid accumulation of lipid, inflammatory cells [Z.A. Fayad et al., Serial, noninvasive, in vivo magnetic resonance microscopy detects the development of atherosclerosis in apolipoprotein E-deficient mice and its progression by arterial wall remodeling, J. Magn. Reson. Imag., 17(2):184-189 (2003)] and friable material on the plaque surface is demonstrated to generate thrombi, which occlude smaller elements of the coronary artery system, downstream. Autopsy studies show that atheroma develops from a very early age. Identification of vulnerable plaque *in vivo* would provide a means of early identification of subjects at risk and a non-invasive means of monitoring preventive therapies. Current technologies (e.g., coronary angiography) observe vessel lumen, not the wall, and hence offer little guidance on the nature of plaque. Occlusion, detected in CT spiral, EBT and other non-invasive methods is misleading. A.P. Lin et al.' Reduced glutamate neurotransmission in patients with Alzheimer's disease - an in vivo 13C magnetic resonance spectroscopy study MAGMA, 16:29-42 (2003), discloses MRI of patients with Alzheimer's disease using ¹³C-glucose. Hence, there is a need of a fast and efficient technology to detect vulnerable plaque *in vivo.* There is therefore a need in the art, for instance, for a PASADENA precursor molecule that binds to atherosclerotic plaque and permits rapid detection of vulnerable plaque in coronary vessels of the smallest caliber, *in vivo.*

Along these lines, there remains a strong need in the art for improved reporter molecules for use in connection with MR imaging for a wide range of diseases and physiologic conditions.

### SUMMARY OF THE INVENTION

In one embodiment, the invention includes a method of magnetic resonance imaging of a subject, comprising: exposing a subject that has been administered with a contrast agent prepared by reacting parahydrogen enriched hydrogen with a hydrogenatable magnetic resonance imaging agent precursor or substrate comprising a non- hydrogen non-zero nuclear spin nucleus, wherein the contrast agent is adapted to target a complementary substance in the subject, to radiation of a frequency selected to excite nuclear spin transitions of the non-zero nuclear spin nucleus in the contrast agent; and detecting magnetic resonance signals of the non-zero nuclear spin nucleus from the subject. The non-zero nuclear spin nucleus may be ¹³C. The subject may be a mammal. Following administration, the contrast agent may biochemically interact with the complementary substance. The contrast agent may comprise ¹³C-stilbamidine. The complementary substance may be selected from the group consisting of an amyloid plaque, a β-amyloid plaque, acetylcholinesterase, and combinations thereof. The precursor or substrate may be selected from the group consisting of hyper-polarized succinate, hyper-polarized diphenylacetylene, hyper-polarized stilbene, hyper-polarized glucose, hyper-polarized dehydroglucose, phosphoenol pyruvate, hyper-polarized fumarate, hyper-polarized succinate, hyper-polarized glutamate, hyper-polarized precursor of choline, hyper-polarized precursor of curcumin, the hyper-polarized compound of Formula I: 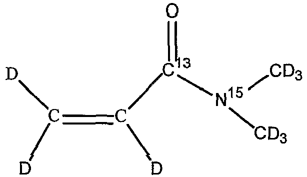 the hyper-polarized compound of Formula II (2,2,3,3-tetraflroro-propyl acrylate ("TFPA")): 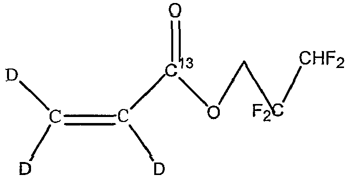 the hyper-polarized compound of Formula III: 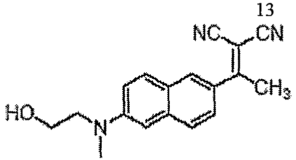 and combinations thereof. The precursor or substrate may be hyper-polarized dehydroglucose and the complementary substance may be neurons. The precursor or substrate may be phosphoenol pyruvate and the complementary substance may be leukocytes. The precursor or substrate may be hyper-polarized fumarate, hyper-polarized succinate or a combination thereof and the complementary substance may be neurons. The precursor or substrate may be hyper-polarized glutamate and the complementary substance may be immune cells. The precursor or substrate may be the hyper-polarized compound of Formula I and the complementary substance may be immune cells. The precursor or substrate may be the hyper-polarized compound of Formula II and the complementary substance may be atherosclerotic plaque and/or stem cells.

In another embodiment, the invention includes a kit for magnetic resonance imaging, comprising: a contrast agent prepared by reacting parahydrogen enriched hydrogen with a hydrogenatable magnetic resonance imaging agent precursor or substrate comprising a non-hydrogen non-zero nuclear spin nucleus, wherein the contrast agent is adapted to target a complementary substance in a subject; and instructions to administer the contrast agent to the subject, expose the subject to radiation of a frequency selected to excite nuclear spin transitions of the non-zero nuclear spin nucleus in the contrast agent, and detect magnetic resonance signals of the non-zero nuclear spin nucleus from the subject. The non-zero nuclear spin nucleus may be ¹³C. The subject may be a mammal. Following administration, the contrast agent may biochemically interact with the complementary substance. The contrast agent may comprise ¹³C-stilbamidine. The complementary substance may be selected from the group consisting of an amyloid plaque, a β-amyloid plaque, acetylcholinesterase, and combinations thereof. The precursor or substrate may be selected from the group consisting of hyper-polarized succinate, hyper-polarized diphenylacetylene, hyper-polarized stilbene, hyper-polarized glucose, hyper-polarized dehydroglucose, phosphoenol pyruvate, hyper-polarized fumarate, hyper-polarized succinate, hyper-polarized glutamate, hyper-polarized precursor of choline, hyper-polarized precursor of curcumin, the hyper-polarized compound of Formula I: 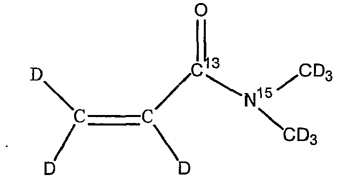 the hyper-polarized compound of Formula II (2,2,3,3-tetraflroro-propyl acrylate ("TFPA")): 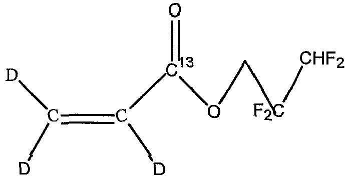 the hyper-polarized compound of Formula III: 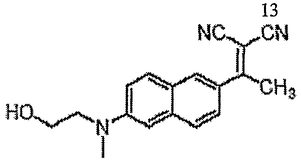 and combinations thereof.

In another embodiment, the invention includes a contrast agent comprising ¹³C-stilbamidine for use in a method of diagnosing and/or prognosing Alzheimer's disease in a mammal, comprising: administering to the mammal a contrast agent comprising ¹³C-stilbamidine prepared by reacting parahydrogen enriched hydrogen with diphenylacetylene, wherein the contrast agent is adapted to target acetylcholinesterase, amyloid plaque and/or β-amyloid plaque in the mammal; exposing the mammal to radiation of a frequency selected to excite nuclear spin transitions of a non-zero nuclear spin nucleus in the contrast agent; detecting magnetic resonance signals of the non-zero nuclear spin nucleus from the mammal; and diagnosing and/or prognosing Alzheimer's disease in the mammal based on the magnetic resonance signals.

In another embodiment, the invention includes a medical device for use in connection with magnetic resonance imaging, comprising: a medical device; and a quantity of a contrast agent impregnated in or coated upon the medical device, wherein the contrast agent is prepared by reacting parahydrogen enriched hydrogen with a hydrogenatable magnetic resonance imaging agent precursor or substrate comprising a non-hydrogen non-zero nuclear spin nucleus, and the contrast agent is adapted to target a complementary substance in a mammal. The non-zero nuclear spin nucleus may be ¹³C. The precursor or substrate may be diphenylacetylene, the contrast agent may comprise ¹³C-stilbamidine, and the complementary substance may be selected from the group consisting of an amyloid plaque, a β-amyloid plaque, acetylcholinesterase, and combinations thereof. The precursor or substrate may be selected from the group consisting of hyper-polarized succinate, hyper-polarized diphenylacetylene, hyper-polarized stilbene, hyper-polarized glucose, hyper-polarized dehydroglucose, phosphoenol pyruvate, hyper-polarized fumarate, hyper-polarized succinate, hyper-polarized glutamate, hyper-polarized precursor of choline, hyper-polarized precursor of curcumin, the hyper-polarized compound of Formula I: 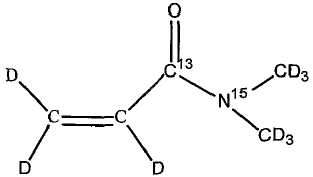 the hyper-polarized compound of Formula II (2,2,3,3-tetraflroro-propyl acrylate ("TFPA")): 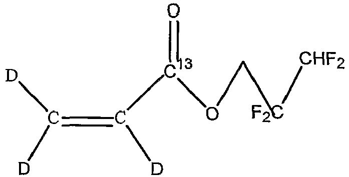 the hyper-polarized compound of Formula III: 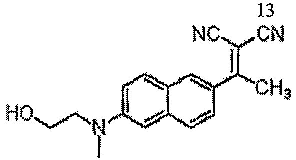 and combinations thereof.

Other features and advantages of the invention will become apparent from the following detailed description, taken in conjunction with the accompanying figures, which illustrate, by way of example, various features of embodiments of the invention.

### BRIEF DESCRIPTION OF THE FIGURES

Exemplary embodiments are illustrated in referenced figures.

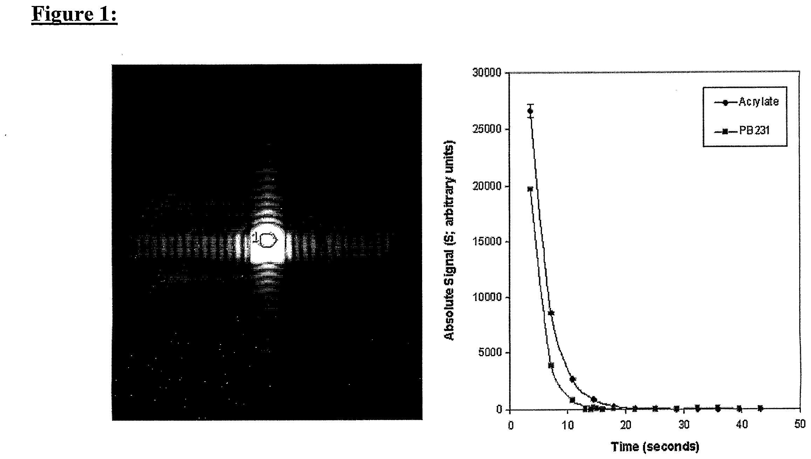

Figure 1 depicts sub-second ¹³C image of 3ml syringe of hyperpolarized ¹³C substrate, in accordance with an embodiment of the present invention. Decay of ¹³C hyperpolarized signal over time. Time courses of ¹³C signal decline measured in ¹³C hydroxyethylpropionate and sodium succinate in arbitrary units (a.u.) were similar

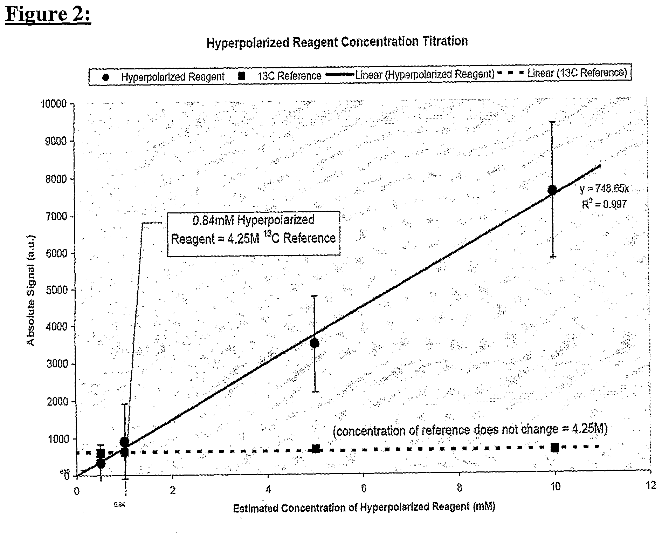

Figure 2 depicts hyperpolarized reagent concentration titration, in accordance with an embodiment of the present invention. 3ml syringes of titrated concentrations of hyperpolarized ¹³C substrate were imaged in the surface coil with a 4.25M phantom of 1-¹³C labeled acetate to serve as a signal and spatial reference phantom. A signal as low as 0.1mM was detected. At a concentration of 0.64mM, the absolute signal of the PASADENA reagent was equivalent to 4.25M of ¹³C signal from the reference phantom, thereby confirming signal enhancement of over 6,000 times.

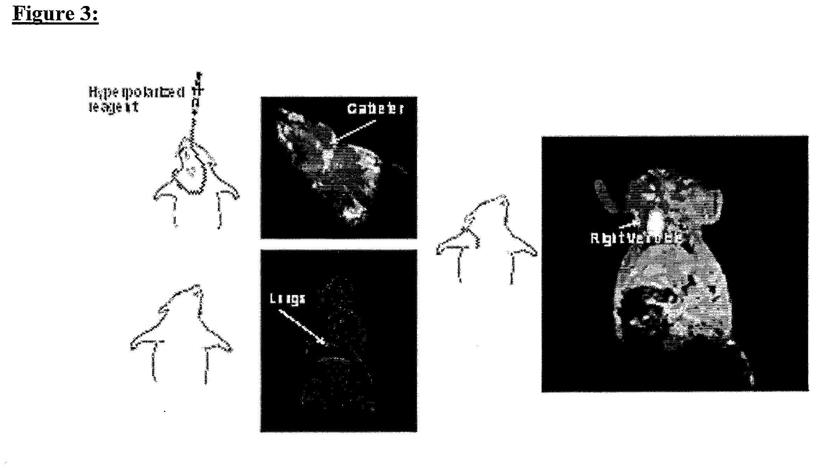

Figure 3 depicts real time 3D ¹³C *in vivo* rodent imaging with PASADENA, in accordance with an embodiment of the present invention. Hyperpolarized ¹³C imaging reagents were administered via jugular vein, and the resulting ultra fast ¹³C MR images displayed as overlay on proton images of the same animal. The advantage of 3D FIESTA imaging is the ability to reconstruct 3D images as shown in A) images of the catheter, as well as several slices in the same experiment thereby demonstrating enhancement of the B) lungs and C) heart.

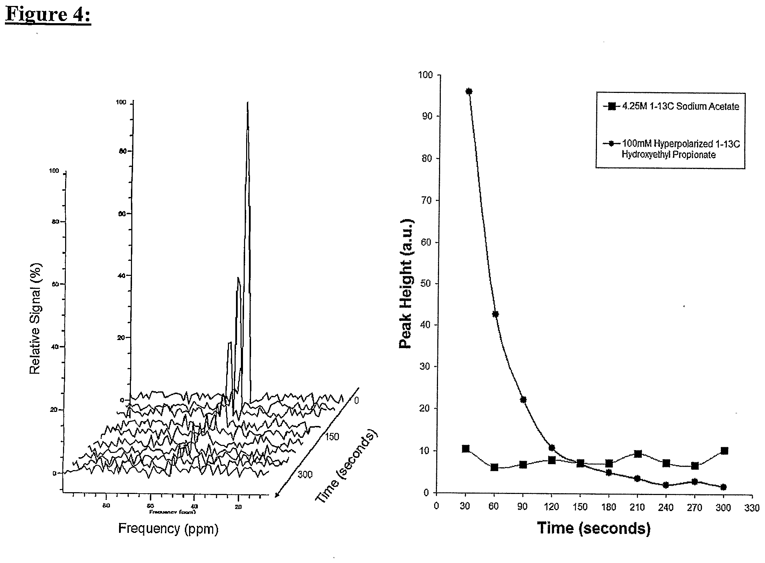

Figure 4 depicts time course of ¹³C CSI of hyperpolarized ¹³C reagent, in accordance with an embodiment of the present invention. ¹³C CSI readily demonstrates acquisition of the hyperpolarized signal with correct chemical shift (left) and demonstrable increase in SNR (right).

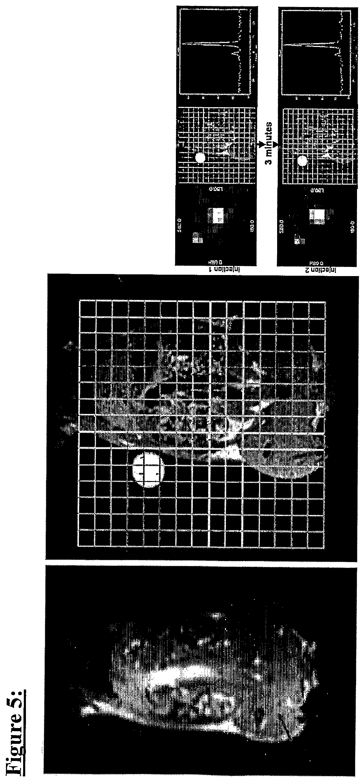

Figure 5 depicts, in accordance with an embodiment of the present invention, left: ¹³C MRI of hyperpolarized ¹³C reagent injected into a cannula placed at the entrance of the femoral artery. The vena cava of the rat can be seen enhanced in the ¹³C images. Middle: ¹³C CSI of the same experiment. The CSI grid is overlaid over ¹H image demonstrating accurate localization of the CSI data. CSI provides the additional information of chemical shift. Right: ¹³C CSI acquired with 1^{st} injection (top) and 2^{nd} injection three minutes later (bottom). These results demonstrate that not only can morphological and chemical information be captured in various embodiments of the present invention, but dynamic time series information can be captured as well.

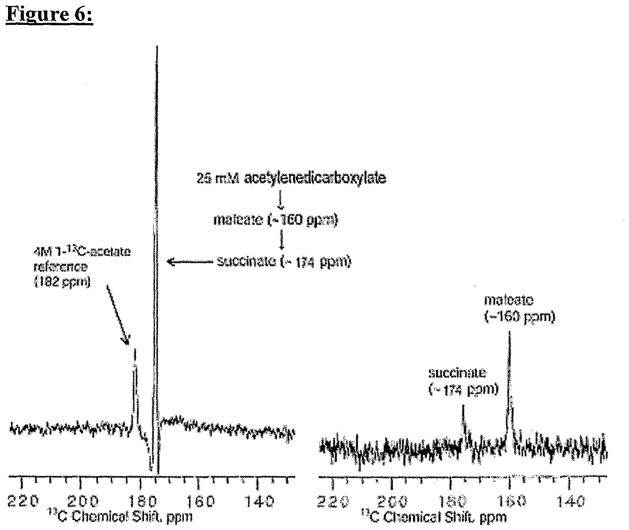

Figure 6 depicts, in accordance with an embodiment of the present invention, (A) ¹³C NMR spectrum of hyperpolarized 1-¹³C-succinate from Acetylene dicarboxylate acquired at 4.7T. 2.8 mL solution of 4.4M 1-¹³C-acetate is used as a reference at ∼182 ppm. A single transient ¹³C NMR spectrum revealed ∼4,400 fold signal enhancement with respect to Boltzman polarization. (B) ¹³C spectrum of the post-hyperpolarized reaction mixture at Boltzman polarization, 128 transients.

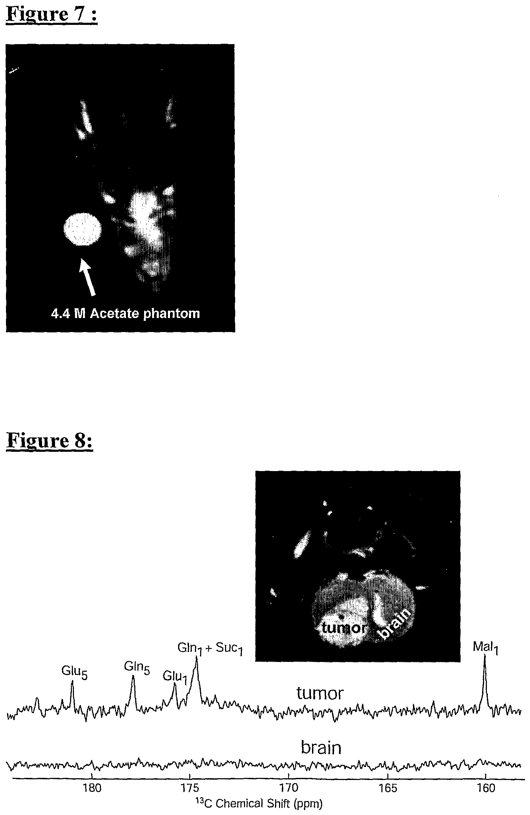

Figure 7 depicts *In Vivo* ¹³C 3D FIESTA imaging of rat brain with PASADENA at 1.5T, in accordance with an embodiment of the present invention. Carotid arterial injection of 25 mM, 1.5 mL hyperpolarized ¹³C succinate in a rat.
Figure 8 depicts *Ex vivo* MAS ¹³C spectra of brain (lower) and brain tumor tissues (upper), in accordance with an embodiment of the present invention. 80 mg of tissue was used in each experiment. Representative *in vivo* T2 weighted image of 9L tumor bearing brain is shown. ¹³C precursors, succinate and maleate, as well as putative products of tumor metabolism, glutamine (Gln) and glutamate (Glu) are assigned based on model solutions. Note the absence of ¹³C enrichment in normal brain tissue from the same animal.

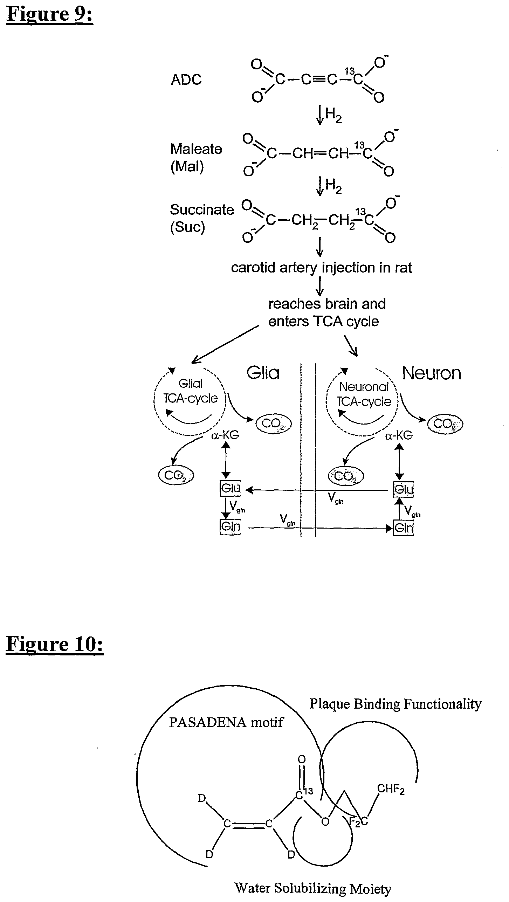

Figure 9 depicts a diagram of an experimental design, in accordance with an embodiment of the present invention. 25 mM aqueous solution of Acetylene dicarboxylate is hydrogenated in the polarizer to yield 3 mL of maleate and succinate products, which is then injected in the carotid artery of 9L tumor bearing rat. It is believed that the hydrogenated products reach the brain and enter glial and neuronal TCA cycle to yield glutamine and glutamate as final product *in vivo.*
Figure 11 depicts a PASADENA precursor, in accordance with an embodiment of the present invention.

### DETAILED DESCRIPTION OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994); March, Advanced Organic Chemistry Reactions, Mechanisms and Structure 4th ed., J. Wiley & Sons (New York, NY 1992); and Sambrook and Russel, Molecular Cloning: A Laboratory Manual 3rd ed., Cold Spring Harbor Laboratory Press (Cold Spring Harbor, NY 2001), provide one skilled in the art with a general guide to many of the terms used in the present application.

One skilled in the art will recognize many methods and materials similar or equivalent to those described herein, which could be used in the practice of the present invention. For purposes of the present invention, the following terms are defined below.

"Mammal" as used herein refers to any member of the class *Mammalia,* including, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs. The term does not denote a particular age or sex. Thus, adult and newborn subjects, as well as fetuses, whether male or female, are intended to be included within the scope of this term.

The invention is based upon the use of magnetic resonance ("MR") imaging together with hyperpolarized substances to present a MR-signal only from hyperpolarized nucleus and from the tissue or vessel where the nucleus may enter. By way of example, the calculated times and concentrations when one particular contrast agent, developed in accordance with an embodiment of the present invention, reaches different parts of the human body is illustrated in Figure 6. More generally, the invention relates to the use of the PASADENA polarization technique to prepare contrast agents or reporter molecules for MR imaging. There are at least two functional aspects of the inventive contrast agent technology that are believed to represent improvements over conventional agents: (1) targeting complementary molecules in the body, and (2) processing through predetermined, natural metabolic pathways to produce reporter molecules. It is believed that PASADENA polarization has not previously been employed in the art in connection with these biochemically functional approaches.

In one embodiment, an inventive reporter molecule targets at least one complementary molecule in the body. Conventional reporter molecules simply disperse within the body (*e.g*., throughout the cardiovascular circulation). However, in one embodiment, the reporter molecules of the present invention are configured to biochemically interact with and thereby target (*e.g.,* bind to) specific molecules *in vivo*. One of skill in the art can readily employ a PASADENA polarization technique in connection with a molecule of interest to prepare a contrast agent that behaves in this manner, and that is targeted to a specific molecule or group of molecules.

By way of example, in one particular embodiment of the invention, the inventors have identified a stilbene precursor (diphenylacetylene) that, when appropriately enriched with stable-isotope ¹³C in conjunction with PASADENA polarization, produces a reporter molecule (¹³C-stilbamidine) suitable for use as a reporter molecule in connection with various embodiments of the present invention. While not wishing to be bound by any particular theory, the ¹³C-stilbamidine molecule is believed to possess several characteristics important for MR imaging, especially in connection with Alzheimer's disease diagnosis and treatment, and in other physiologic conditions in which the nature and extent of plaque in the brain is relevant. It is believed that this molecule crosses the blood-brain barrier with relative ease; something which is not possible with many commercially available reporter molecules of larger molecular size. While not wishing to be bound by any particular theory, it is further believed that the ¹³C-stilbamidine molecule targets two items relevant to Alzheimer's pathology: (1) the acetylcholine receptor, acetylcholinesterase (acetylcholine is a neurotransmitter known to be deficient in certain areas of the brain in Alzheimer's patients), and (2) β-amyloid plaque (individuals with Alzheimer's disease produce amyloidogenic peptides in the brain, resulting in the formation of amyloid plaques that are believed to be one of the disease's fundamental problems). The ¹³C-stilbamidine molecule is believed to impart dramatic ¹³C MR imaging enhancement; achieving as much as a 10,000-fold increase in sensitivity as compared with available products.

In another embodiment, the present invention relates to reporter molecules that, once administered to a subject (*e.g*., a mammal), are processed through a predetermined, natural metabolic pathway to produce one or more molecules that themselves become the contrast agent for MR imaging. This aspect of the invention is based on the fact that such metabolic processing does not quench the polarization of the reporter molecule; rather, the polarization may be preserved through the metabolic process, and the downstream reporter molecules thereby remain suitable for use in connection with MR imaging. One of skill in the art can readily identify suitable molecules that may be used in connection with this aspect of the present invention; such molecules may be selected based upon, among other things, the metabolic pathway of interest and the capacity of the molecule to preserve its polarization through the metabolic process.

A wide range of reporter molecules produced with the aforementioned PASADENA technique are useful in connection with alternate embodiments of the present invention, as will be readily recognized by those of skill in the art. Some of these molecules are described in U.S. Patent No. 6,574,495, and, in connection with the present invention, may be selected or further modified to implement or embody the functional features described above.

In accordance with an embodiment of the present invention, molecules are identified to introduce para-hydrogen in biological systems. Desirable qualities of precursors to these contrast agents may include one or more of the following: (1) an unsaturated bond suitable for hydrogenation by molecular addition; (2) the time scale of the hydrogenation reaction is shorter than the spin lattice relaxation times of the intermediates and products; (3) enriched ¹³C/¹⁵N nucleus with scalar coupling to the added protons; (4) water solubility and non-toxicity to mammals (e.g. humans); (5) may be introduced into a specific biological system safely and quickly before appreciable spin lattice relaxation; (6) is easily obtainable or can be synthesized in a cost effective manner. By way of example, Table 1 illustrates several precursor molecules along with their substrate molecules (and anticipated biomolecular targets) in accordance with various embodiments of the present invention.

**Table 1**

| PASADENA Precursors | Substrate Molecules (Biomolecular Target) |
|---|---|
| 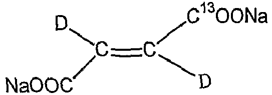 | Hyper-polarized Succinate (Neuron) |
| 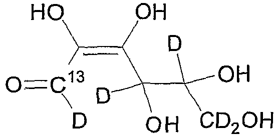 | Hyper-polarized Glucose (Neuron) |
| 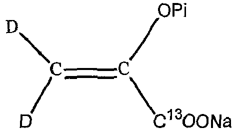 | Phosphoenol pyruvate (Leukocyte) |
| 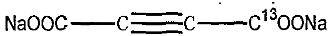 | Hyper-polarized Fumarate, Succinate (Neuron) |
| 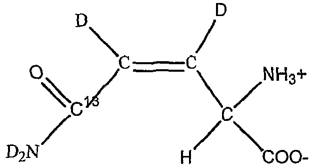 | Hyper-polarized Glutamate (Immune Cells) |
| 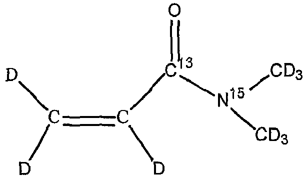 | Hyper-polarized N¹⁵ precursor (Immune Cells) |
| 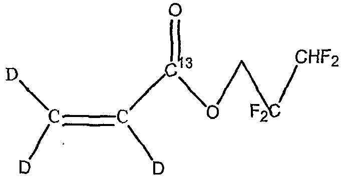 | Hyper-polarized reagent (Atherosclerotic Plaque, Stem Cells) |
| 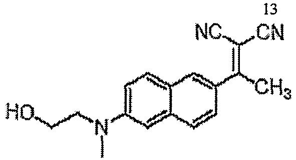 | Hyper-polarized precursor (Chemical marker for Alzheimer's Disease) |
| 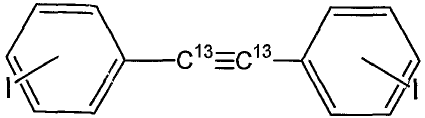 | Hyper-polarized functionalized Stilbene Analog family (Chemical marker of amyloid plaques; Alzheimer's Disease) |
| 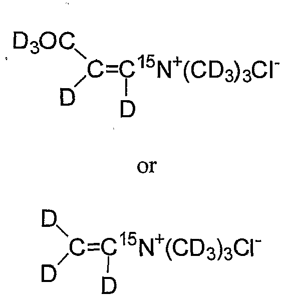 | Hyper-polarized precursor of choline (Cancer marker) |
| 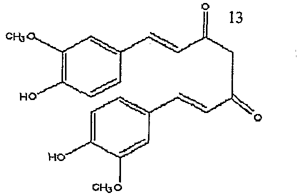 | Hyper-polarized precursor of curry powder (curcumin) (Chemical marker for Alzheimer's Disease) |

The present invention is also directed to methods of imaging various substances in the body by providing a quantity of a reporter molecule or reporter molecule precursor, introducing a sufficient quantity thereof into a subject, and thereafter imaging a target substance. In various embodiments of the present invention; the aforementioned methods of imaging may be used in connection with a range of applications, including, the diagnosis/prognosis of disease or other physiological conditions and the study of biological systems. In various embodiments, the contrast agent compositions utilized by the present inventive methods may include a pharmaceutically acceptable excipient. "Pharmaceutically acceptable excipient" means an excipient that is useful in preparing a pharmaceutical composition that is generally safe, non-toxic, and desirable, and includes excipients that are acceptable for veterinary use as well as for human pharmaceutical use. Such excipients may be solid, liquid, semisolid, or, in the case of an aerosol composition, gaseous.

In various embodiments, the contrast agent compositions according to the invention may be formulated for delivery via any route of administration. "Route of administration" may refer to any administration pathway known in the art, including aerosol, nasal, oral, transmucosal, transdermal or parenteral. "Parenteral" refers to a route of administration that is generally associated with injection, including intraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal. Via the parenteral route, the contrast agent compositions may be in the form of solutions or suspensions for infusion or for injection, or as lyophilized powders.

The compositions according to the invention can also contain any pharmaceutically acceptable carrier. "Pharmaceutically acceptable carrier" as used herein refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or a combination thereof. Each component of the carrier must be "pharmaceutically acceptable" in that it must be compatible with the other ingredients of the formulation. It must also be suitable for use in contact with any tissues or organs with which it may come in contact, meaning that it must not carry a risk of toxicity, irritation, allergic response, immunogenicity, or any other complication that excessively outweighs its therapeutic benefits.

The contrast agent compositions according to the invention can also be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline, alcohols and water. Solid carriers include starch, lactose, calcium sulfate, dihydrate, terra alba, magnesium stearate or stearic acid, talc, pectin, acacia, agar or gelatin. The carrier may also include a sustained release material such as glyceryl monostearate or glyceryl distearate, alone or with a wax.

In addition to the PASADENA technique, the contrast agent compositions are made following the conventional techniques of pharmacy involving milling, mixing, granulation, and compressing, when necessary, for tablet forms; or milling, mixing and filling for hard gelatin capsule forms. When a liquid carrier is used, the preparation will be in the form of a syrup, elixir, emulsion or an aqueous or nonaqueous suspension. Such a liquid formulation may be administered directly p.o. or filled into a soft gelatin capsule.

The contrast agent compositions according to the invention may be delivered in an amount sufficient to effect their purpose. The precise therapeutically effective amount is that amount of the composition that will yield the most effective results in terms of efficacy of imaging in a given subject. This amount will vary depending upon a variety of factors, including the characteristics of the reporter molecule (including activity, pharmacokinetics, pharmacodynamics, and bioavailability), the physiological condition of the subject (including age, sex, disease type and stage, general physical condition, responsiveness to a given dosage, and type of reporter molecule), the nature of the pharmaceutically acceptable carrier or carriers in the formulation, and the route of administration. One skilled in the clinical and pharmacological arts will be able to determine an effective amount through routine experimentation, for instance, by monitoring a subject's response to administration of a compound and adjusting the dosage accordingly. For additional guidance, see Remington: The Science and Practice of Pharmacy (Gennaro ed. 20th edition, Williams & Wilkins PA, USA) (2000).

The present invention is also directed to a kit for use in connection with MR imaging. The kit is an assemblage of materials or components, including at least one of the inventive contrast agent compositions; more specifically, a composition comprising a quantity of at least one of the inventive reporter molecules, as described above.

The exact nature of the components configured in the inventive kit depends on the kit's intended purpose. For example, some embodiments are configured for the purpose of diagnosing specific diseases, while others are useful in connection with studying biological processes unrelated to healthcare needs (*e.g*., studying metabolic pathways). In one embodiment, the kit is configured particularly for the purpose of diagnosing or prognosing a medical condition in a mammalian subject. In another embodiment, the kit is configured particularly for the purpose of diagnosing or prognosing human subjects.

Instructions for use may be included in the kit. "Instructions for use" typically include a tangible expression describing the technique to be employed in using the components of the kit to effect a desired outcome, such as to diagnose or prognose a physiological condition. In connection with various embodiments of the present invention, the instructions for use may include instructions to administer a quantity of an inventive reporter molecule to a mammalian subject, and to then implement a MR imaging technique to obtain diagnostic or prognostic information about the subject.

Optionally, the kit also contains other useful components, such as, diluents, buffers, pharmaceutically acceptable carriers, syringes, catheters, applicators, pipetting or measuring tools, bandaging materials or other useful paraphernalia as will be readily recognized by those of skill in the art.

The materials or components assembled in the kit can be provided to the practitioner stored in any convenient and suitable ways that preserve their operability and utility. For example the components can be in dissolved, dehydrated, or lyophilized form; they can be provided at room, refrigerated or frozen temperatures. The components are typically contained in suitable packaging material(s). As employed herein, the phrase "packaging material" refers to one or more physical structures used to house the contents of the kit, such as inventive compositions. The packaging material is constructed by well known methods, preferably to provide a sterile, contaminant-free environment. As used herein, the term "package" refers to a suitable solid matrix or material such as glass, plastic, paper, foil, capable of holding the individual kit components. Thus, for example, a package can be a glass vial used to contain suitable quantities of an inventive composition including a quantity of one or more reporter molecules. The packaging material generally has an external label which indicates the contents and/or purpose of the kit and/or its components.

Still further embodiments of the present invention are directed to drug discovery technology using the inventive contrast agent compositions. The contrast agent compositions of the invention may be used, for example, in connection with animal trials of novel therapeutic compounds to study the compounds' efficacy or other physiological properties of interest in screening drug candidates or in amassing otherwise valuable information about a particular therapeutic compound. Techniques and protocols for doing so will be readily ascertainable by those of skill in the art, and can be implemented without undue experimentation.

In a still further, alternate embodiment of the invention, a ¹³C-PASADENA reporter molecule of the present invention may be incorporated in a catheter or other medical device or instrument, resulting in a signal from the catheter, device or instrument (as used herein, each of these terms is within the scope of "medical device"). This may provide a method of *in vivo* visualization of the medical device containing the hyperpolarized reporter molecule. There are a wide variety of applications of such an embodiment of the instant invention, including, placement and/or positioning of such medical devices in a mammal (e.g., during a surgical procedure). The inventive reporter molecule may be incorporated into a medical device by known techniques, such as by impregnating and/or coating at least a portion of the material used to construct the medical device with a quantity of the reporter molecule.

### EXAMPLE

### Preparation of Reporter Molecule for Imaging Vulnerable Plaques

In one embodiment of the present invention, a PASADENA precursor is synthesized with commercially available starting materials: hydroxyethylacrylate, glycerol, and fluoro-alcohols. Initial synthesis is carried out by non-enriched starting materials to, *e.g*., reduce the cost of overall synthesis. The precursor may be particularly effective at imaging vulnerable plaques, based on its biomolecular interaction with the same, and may have the chemical formula illustrated in Figure 11.

To reach the low temperatures required for obtaining a large fraction of para-hydrogen, a 3 kW closed-loop helium cryo-cooler may be used. Para-hydrogen may be produced by passing normal hydrogen gas through a catalyst at a temperature of about 14 K. The production rate may be about one seven liter cylinder filled to a pressure of about 30 bar in one hour. The resulting gas may consist of more than about 95% para-hydrogen, and may thereafter be used in a hydrogenation reaction to produce an imaging agent.

Para-hydrogen gas may be used in a chemical reaction (hydrogenation) to produce a contrast agent in connection with an embodiment of the present invention. In order to preserve the spin correlation between the protons immediately after hydrogenation (Figure 3), a catalyst, such as a rhodium catalyst, may be used to transfer the protons as a unit onto the substrate, without scrambling. Removal of toxic rhodium may be desirable for making the inventive technique amenable for biomedical applications. Cation and anion exchange resins may be used to filter out the rhodium complexes from the solution immediately before injecting into biological systems (*i.e.,* below OSHA Permissible Exposure Limit of 0.1 mg/m³). The chemistry may take place at a temperature of about 60°C in a reactor where a solution containing both substrate and catalyst is injected, during about two seconds, into an atmosphere of para-hydrogen gas.

As will be appreciated by one of skill in the art, if a substrate being hydrogenated contains N nuclei with non-zero spin, the resulting density operator for the total spin system after hydrogenation with para-hydrogen may be quite different from that which is obtained for the same system at thermal equilibrium. The density operator of the substrate can be approximated by the unity operator, and the total density operator may be given by the direct (tensor) product with the density operator of para-hydrogen. Because the total density operator may not be diagonal in the base of eigenstates of the Hamiltonian, the off-diagonal elements may oscillate as a function of time. This may result in most cases to a vanishing of the off-diagonal elements and hence a modification of the density operator.

Without further treatment the spin system is not suitable for imaging since the nuclear MR spectrum exhibits peaks which have an anti-phase relationship. The spin order of the protons must first be converted into polarization of the carbon nuclei. A reactor may be placed inside a solenoid coil with a magnetic field strength close to about 1.76 mT. An untuned saddle coil for transmitting linearly polarized RF pulses may be placed between the solenoid coil and the reactor. The B1 intensity may correspond to a 90 degree proton (square) pulse of 0.0879 ms and a corresponding carbon pulse of 0.1724 ms. Pulses may be generated in MATLAB, and sampled at a frequency of 300 kHz. The Larmor frequencies of carbon and proton are 19 kHz and 75 kHz respectively at this field.

The pulse sequence may be initiated as soon as the hydrogenation process starts, with a proton decoupling that lasts for about 4 seconds. The purpose of the proton decoupling is to preserve the spin correlation between the protons, thus preventing the cancellation of the off-diagonal terms in the density operator. The gain is two-fold. First, the protons are forced to remain in the singlet state so their mutual dipolar relaxation is reduced. Second, this state has lower entropy, which makes it possible to obtain a larger polarization for the carbon nuclei.

The delay times (*e.g*., three) used in the pulse sequence depend on the mutual scalar couplings between the protons and the carbon. The pulse sequence, after the proton decoupling, can be divided into, *e.g*., two parts. The first part may include two evolution periods divided by a 180 degree pulse on carbon that transforms the density operator into an intermediate form. The second part may transform this intermediate form, using an initial 90 degree pulse in quadrature and a subsequent evolution period, into a transverse carbon magnetization along the y-axis. A final 90 degree pulse along the x-axis produces the desired longitudinal magnetization. The theoretical carbon polarization, assuming hydrogenation with pure para-hydrogen and neglecting relaxation, is 98.6 % using this pulse sequence.

The refocusing pulses, simultaneous on both proton and carbon to allow for evolution of scalar couplings, may be simple square pulses, whereas the proton decoupling pulses and the remaining carbon pulses may be broad band pulses with, e.g., both a homogeneous excitation and good phase control. All equipment may be built into a single temperature controlled cabinet, divided into compartments where the different steps in the process take place (Figure 4). The valves and the RF pulses may be controlled by computer, using software (*e.g*., written in LabView). A ¹³C polarization equal to 44% may be obtained, which is approximately half of the polarization predicted by theory (Figure 5). This discrepancy is owing to both relaxation and a lack of complete phase control of the RF pulses.

## Claims

1. A method of magnetic resonance imaging of a subject, comprising:
exposing a subject, that has been administered with a contrast agent prepared by reacting parahydrogen enriched hydrogen with a hydrogenatable magnetic resonance imaging agent precursor or substrate comprising a non-hydrogen non-zero nuclear spin nucleus, wherein the contrast agent is adapted to target a complementary substance in the subject,
to radiation of a frequency selected to excite nuclear spin transitions of the non-zero nuclear spin nucleus in the contrast agent; and
detecting magnetic resonance signals of the non-zero nuclear spin nucleus from the subject,
wherein the precursor or substrate is selected from the group consisting of hyper-polarized succinate, hyper-polarized diphenylacetylene, hyper-polarized stilbene, hyper-polarized glucose, hyper-polarized dehydroglucose, phosphoenol pyruvate, hyper-polarized fumarate, hyper-polarized glutamate, 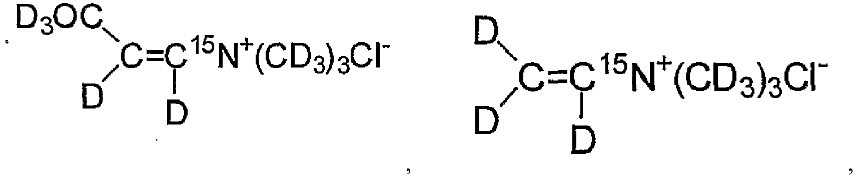 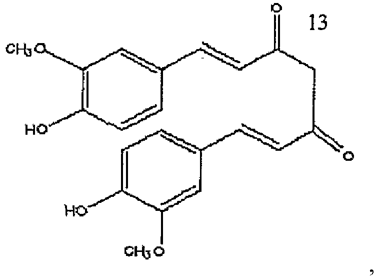 the hyper-polarized compound of Formula I: 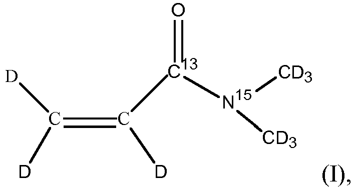 the hyper-polarized compound of Formula II (2,2,3,3-tetraflroro-propyl acrylate ("TFPA")): 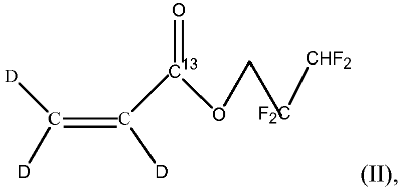 the hyper-polarized compound of Formula III: 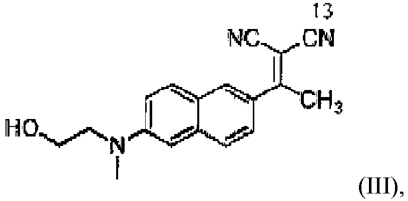 and combinations thereof.

2. The method of claim 1, wherein the non-zero nuclear spin nucleus is ¹³C.

3. The method of claim 1, wherein the subject is a mammal.

4. The method of claim 1, wherein following administration the contrast agent biochemically interacts with the complementary substance in the subject.

5. A method of magnetic resonance imaging of a subject, comprising:
exposing a subject, that has been administered with a contrast agent prepared by reacting parahydrogen enriched hydrogen with a hydrogenatable magnetic resonance imaging agent precursor or substrate comprising a non-hydrogen non-zero nuclear spin nucleus, wherein the contrast agent is adapted to target a complementary substance in the subject,
to radiation of a frequency selected to excite nuclear spin transitions of the non-zero nuclear spin nucleus in the contrast agent; and
detecting magnetic resonance signals of the non-zero nuclear spin nucleus from the subject,
wherein the contrast agent is ¹³C-stilbamidine and the precursor or substrate is a hydrogenatable precursor comprising ¹³C.

6. The method of claim 1, wherein the complementary substance is selected from the group consisting of an amyloid plaque, a *β*-amyloid plaque, acetylcholinesterase, and combinations thereof

7. The method of claim 1, wherein the precursor or substrate is hyper-polarized dehydroglucose and the complementary substance is neurons.

8. The method of claim 1, wherein the precursor or substrate is phosphoenol pyruvate and the complementary substance is leukocytes.

9. The method of claim 1, wherein the precursor or substrate is hyper-polarized fumarate, hyper-polarized succinate or a combination thereof and the complementary substance is neurons.

10. The method of claim 1, wherein the precursor or substrate is hyper-polarized glutamate and the complementary substance is immune cells.

11. The method of claim 1, wherein the precursor or substrate is the hyper-polarized compound of Formula I and the complementary substance is immune cells.

12. The method of claim 1, wherein the precursor or substrate is the hyper-polarized compound of Formula II and the complementary substance is atherosclerotic plaque and/or stem cells.

13. A kit for magnetic resonance imaging, comprising:
a contrast agent prepared by reacting parahydrogen enriched hydrogen with a hydrogenatable magnetic resonance imaging agent precursor or substrate comprising a non-hydrogen non-zero nuclear spin nucleus, wherein the contrast agent is adapted to target a complementary substance in a subject; and
instructions to administer the contrast agent to the subject, expose the subject to radiation of a frequency selected to excite nuclear spin transitions of the non-zero nuclear spin nucleus in the contrast agent, and detect magnetic resonance signals of the non-zero nuclear spin nucleus from the subject;
wherein the precursor or substrate is selected from the group consisting of hyper-polarized succinate, hyper-polarized diphenylacetylene, hyper-polarized stilbene, hyper-polarized glucose, hyper-polarized dehydroglucose, phosphoenol pyruvate, hyper-polarized fumarate, hyper-polarized glutamate, 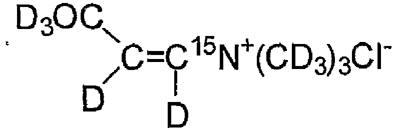 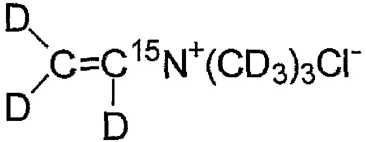 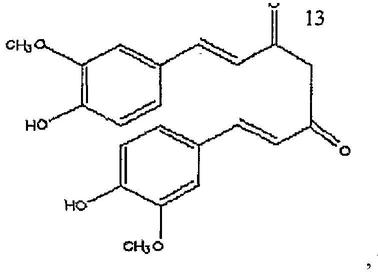 the hyper-polarized compound of Formula I: 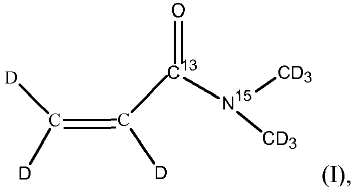 the hyper-polarized compound of Formula II (2,2,3,3-tetraflroro-propyl acrylate ("TFPA")): 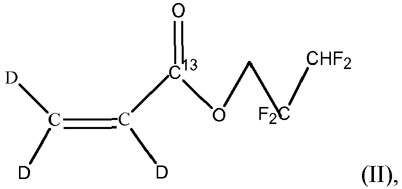 the hyper-polarized compound of Formula III: 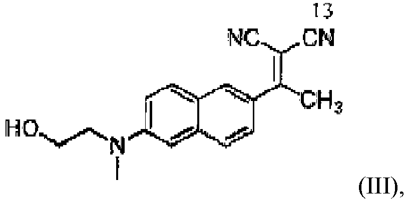 and combinations thereof.

14. The kit of claim 13, wherein the contrast agents is as defined in any one of claims 1, 2 or 4-6.

15. The kit of claim 13, wherein the subject is a mammal.

16. A contrast agent comprising ¹³C-stilbamidine for use in diagnosing and/or prognosing Alzheimer's disease in a mammal,
wherein the contrast agent is adapted for administering to the mammal;
wherein the contrast agent is prepared by reacting parahydrogen enriched hydrogen with diphenylacetylene;
wherein the contrast agent is adapted to target acetylcholinesterase, amyloid plaque and/or β-amyloid plaque in the mammal;
wherein the mammal is adapted to be exposed to radiation of a frequency selected to excite nuclear spin transitions of a non-zero nuclear spin nucleus in the contrast agent;
wherein magnetic resonance signals of the non-zero nuclear spin nucleus is detected from the mammal; and
wherein Alzheimer's disease in the mammal is diagnosed and/or prognosed based on the magnetic resonance signals.

17. A medical device for use in connection with magnetic resonance imaging, comprising:
a medical device; and
a quantity of a contrast agent impregnated in or coated upon the medical device, wherein the contrast agent is prepared by reacting parahydrogen enriched hydrogen with a hydrogenatable magnetic resonance imaging agent precursor or substrate comprising a non-hydrogen non-zero nuclear spin nucleus, and the contrast agent is adapted to target a complementary substance in a mammal;
wherein the precursor or substrate is selected from the group consisting of hyper-polarized succinate, hyper-polarized diphenylacetylene, hyper-polarized stilbene, hyper-polarized glucose, hyper-polarized dehydroglucose, phosphoenol pyruvate, hyper-polarized fumarate, hyper-polarized glutamate, 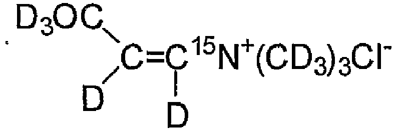 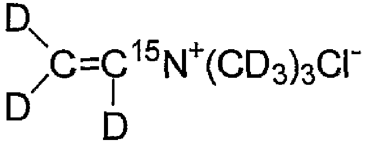 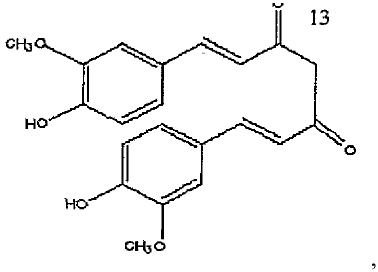 the hyper-polarized compound of Formula I: 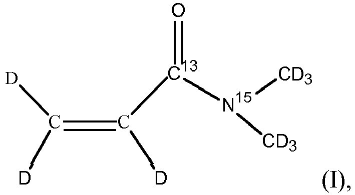 the hyper-polarized compound of Formula II (2,2,3,3-tetraflroro-propyl acrylate ("TFPA")): 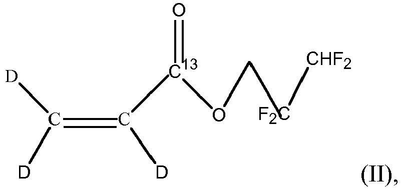 the hyper-polarized compound of Formula III: 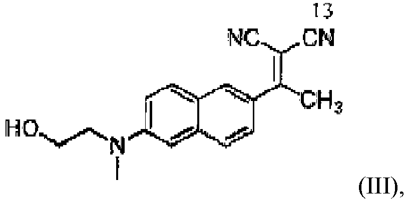 and combinations thereof.

18. The medical device of claim 17, wherein the contrast agent is as defined in claim 2.

19. The medical device of claim 17, wherein the precursor or substrate is diphenylacetylene, the contrast agent comprises ¹³C-stilbamidine, and the complementary substance is selected from the group consisting of an amyloid plaque, a *β*-amyloid plaque, acetylcholinesterase, and combinations thereof.

## Patentansprüche

1. Verfahren zur Magnetresonanztomografie eines Subjekts, umfassend:
Aussetzen eines Subjekts, dem ein Kontrastmittel verabreicht worden ist, das durch Umsetzen von mit Parawasserstoff angereichertem Wasserstoff mit einem hydrierbaren Magnetresonanzabbildungsmittelvorläufer oder - substrat hergestellt wurde, der bzw. das einen Nicht-Wasserstoffkern mit einem Kernspin ungleich null umfasst, wobei das Kontrastmittel dazu angepasst ist, auf eine komplementäre Substanz in dem Subjekt abzuzielen,
einer Strahlung mit einer Frequenz, die ausgewählt ist, um Kernspinübergänge des Kerns mit einem Kernspin ungleich null in dem Kontrastmittel anzuregen; und
Erfassen von Magnetresonanzsignalen des Kerns mit einem Kernspin ungleich null aus dem Subjekt,
wobei der Vorläufer oder das Substrat ausgewählt ist aus der Gruppe bestehend aus hyperpolarisiertem Succinat, hyperpolarisiertem Diphenylacetylen, hyperpolarisiertem Stilben, hyperpolarisierter Glucose, hyperpolarisierter Dehydroglucose, Phosphoenolpyruvat, hyperpolarisiertem Fumarat, hyperpolarisiertem Glutamat, 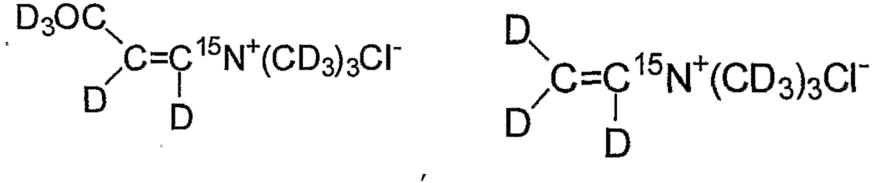 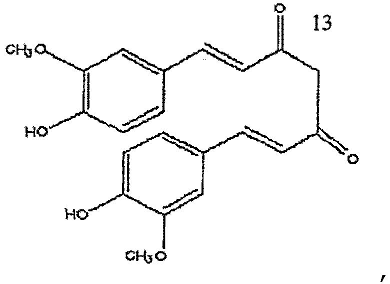 der hyperpolarisierten Verbindung der Formel I: 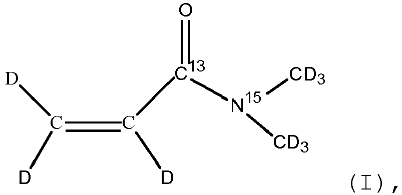 der hyperpolarisierten Verbindung der Formel II (2,2,3,3-Tetraflurorpropylacrylat ("TFPA")): 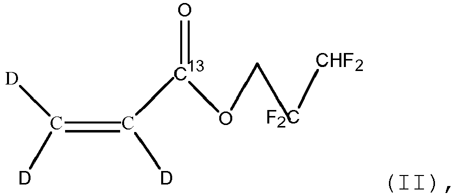 der hyperpolarisierten Verbindung der Formel III: 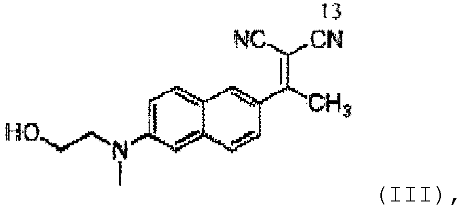 und Kombinationen davon.

2. Verfahren nach Anspruch 1, wobei der Kern mit einem Kernspin ungleich null ¹³C ist.

3. Verfahren nach Anspruch 1, wobei das Subjekt ein Säuger ist.

4. Verfahren nach Anspruch 1, wobei das Kontrastmittel nach der Verabreichung mit der komplementären Substanz in dem Subjekt biochemisch wechselwirkt.

5. Verfahren zur Magnetresonanztomografie eines Subjekts, umfassend:
Aussetzen eines Subjekts, dem ein Kontrastmittel verabreicht worden ist, das durch Umsetzen von mit Parawasserstoff angereichertem Wasserstoff mit einem hydrierbaren Magnetresonanzabbildungsmittelvorläufer oder - substrat hergestellt wurde, der bzw. das einen Nicht-Wasserstoffkern mit einem Kernspin ungleich null umfasst, wobei das Kontrastmittel dazu angepasst ist, auf eine komplementäre Substanz in dem Subjekt abzuzielen,
einer Strahlung mit einer Frequenz, die ausgewählt ist, um Kernspinübergänge des Kerns mit einem Kernspin ungleich null in dem Kontrastmittel anzuregen; und
Erfassen von Magnetresonanzsignalen des Kerns mit einem Kernspin ungleich null aus dem Subjekt,
wobei das Kontrastmittel ¹³C-Stilbamidin ist und der Vorläufer oder das Substrat ein hydrierbarer Vorläufer ist, der ¹³C umfasst.

6. Verfahren nach Anspruch 1, wobei die komplementäre Substanz aus der Gruppe bestehend aus einer Amyloid-Plaque, einer β-Amyloid-Plaque, Acetylcholinesterase und Kombinationen davon ausgewählt ist.

7. Verfahren nach Anspruch 1, wobei der Vorläufer oder das Substrat hyperpolarisierte Dehydroglucose ist und die komplementäre Substanz Neuronen ist.

8. Verfahren nach Anspruch 1, wobei der Vorläufer oder das Substrat Phosphoenolpyruvat ist und die komplementäre Substanz Leukozyten ist.

9. Verfahren nach Anspruch 1, wobei der Vorläufer oder das Substrat hyperpolarisiertes Fumarat, hyperpolarisiertes Succinat oder eine Kombination davon ist und die komplementäre Substanz Neuronen ist.

10. Verfahren nach Anspruch 1, wobei der Vorläufer oder das Substrat hyperpolarisiertes Glutamat ist und die komplementäre Substanz Immunzellen ist.

11. Verfahren nach Anspruch 1, wobei der Vorläufer oder das Substrat die hyperpolarisierte Verbindung der Formel I ist und die komplementäre Substanz Immunzellen ist.

12. Verfahren nach Anspruch 1, wobei der Vorläufer oder das Substrat die hyperpolarisierte Verbindung der Formel II ist und die komplementäre Substanz atherosklerotische Plaque und/oder Stammzellen ist.

13. Kit zur Magnetresonanztomografie, umfassend:
ein Kontrastmittel, das durch Umsetzen von mit Parawasserstoff angereichertem Wasserstoff mit einem hydrierbaren Magnetresonanzabbildungsmittelvorläufer oder - substrat hergestellt wurde, der bzw. das einen Nicht-Wasserstoffkern mit einem Kernspin ungleich null umfasst, wobei das Kontrastmittel dazu angepasst ist, auf eine komplementäre Substanz in einem Subjekt abzuzielen; und
Anweisungen zum Verabreichen des Kontrastmittels an das Subjekt, Aussetzen des Subjekts einer Strahlung mit einer Frequenz, die ausgewählt ist, um Kernspinübergänge des Kerns mit einem Kernspin ungleich null in dem Kontrastmittel anzuregen und Erfassen von Magnetresonanzsignalen des Kerns mit einem Kernspin ungleich null aus dem Subjekt;
wobei der Vorläufer oder das Substrat ausgewählt ist aus der Gruppe bestehend aus hyperpolarisiertem Succinat, hyperpolarisiertem Diphenylacetylen, hyperpolarisiertem Stilben, hyperpolarisierter Glucose, hyperpolarisierter Dehydroglucose, Phosphoenolpyruvat, hyperpolarisiertem Fumarat, 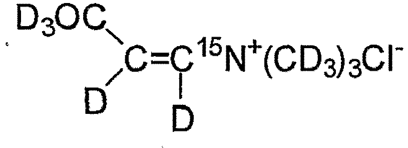 hyperpolarisiertem Glutamat, 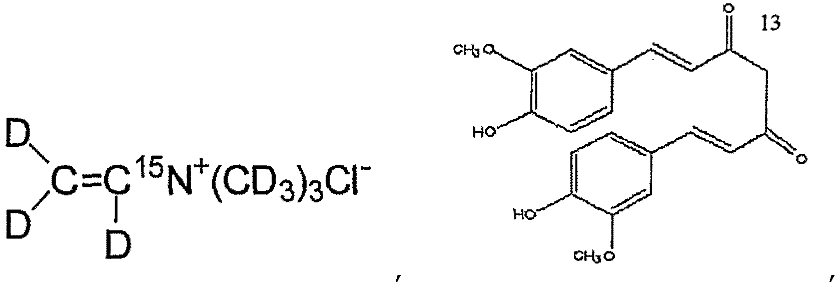 der hyperpolarisierten Verbindung der Formel I: 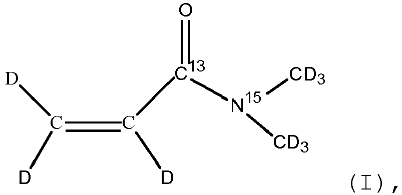 der hyperpolarisierten Verbindung der Formel II (2,2,3,3-Tetraflurorpropylacrylat ("TFPA")): 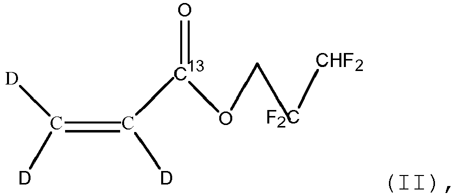 der hyperpolarisierten Verbindung der Formel III: 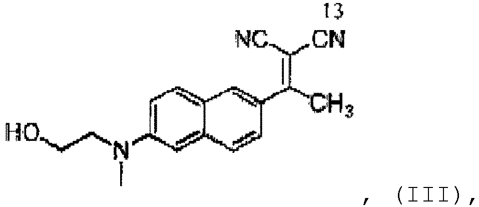 und Kombinationen davon.

14. Kit nach Anspruch 13, wobei die Kontrastmittel wie in einem der Ansprüche 1, 2 oder 4-6 definiert sind.

15. Kit nach Anspruch 13, wobei das Subjekt ein Säuger ist.

16. Kontrastmittel, umfassend ¹³C-Stilbamidin zur Verwendung beim Diagnostizieren und/oder Prognostizieren der Alzheimer-Krankheit bei einem Säuger,
wobei das Kontrastmittel zum Verabreichen an den Säuger angepasst ist;
wobei das Kontrastmittel durch Umsetzen von mit Parawasserstoff angereichertem Wasserstoff mit Diphenylacetylen hergestellt wird;
wobei das Kontrastmittel dazu angepasst ist, auf Acetylcholinesterase, Amyloid-Plaque und/ oder β-Amyloid-Plaque in dem Säuger abzuzielen;
wobei der Säuger dazu angepasst ist, einer Strahlung mit einer Frequenz ausgesetzt zu werden, die ausgewählt ist, um Kernspinübergänge eines Kerns mit einem Kernspin ungleich null in dem Kontrastmittel anzuregen;
wobei Magnetresonanzsignale des Kerns mit einem Kernspin ungleich null von dem Säuger erfasst werden; und
wobei die Alzheimer-Krankheit in dem Säuger auf Grundlage der Magnetresonanzsignale diagnostiziert und/oder prognostiziert wird.

17. Medizinische Vorrichtung zur Verwendung in Verbindung mit Magnetresonanztomografie, umfassend:
eine medizinische Vorrichtung; und
eine Menge eines Kontrastmittels, mit dem die medizinische Vorrichtung imprägniert oder beschichtet ist,
wobei das Kontrastmittel durch Umsetzen von mit Parawasserstoff angereichertem Wasserstoff mit einem hydrierbaren Magnetresonanzabbildungsmittelvorläufer oder - substrat hergestellt wurde, der bzw. das einen Nicht-Wasserstoffkern mit einem Kernspin ungleich null umfasst, wobei das Kontrastmittel dazu angepasst ist, auf eine komplementäre Substanz in einem Säuger abzuzielen,
wobei der Vorläufer oder das Substrat ausgewählt ist aus der Gruppe bestehend aus hyperpolarisiertem Succinat, hyperpolarisiertem Diphenylacetylen, hyperpolarisiertem Stilben, hyperpolarisierter Glucose, hyperpolarisierter Dehydroglucose, Phosphoenolpyruvat, hyperpolarisiertem Fumarat, hyperpolarisiertem Glutamat, 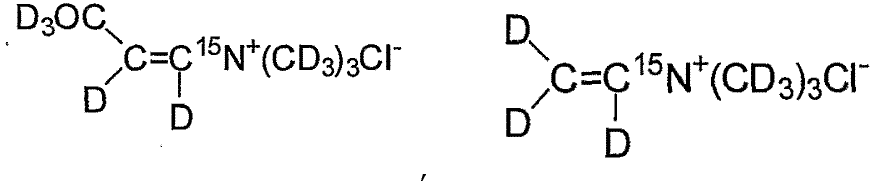 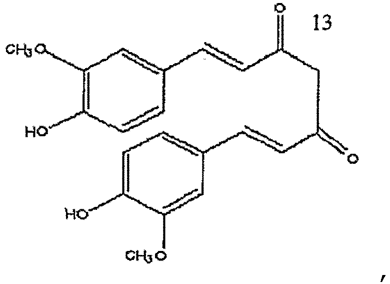 der hyperpolarisierten Verbindung der Formel I: 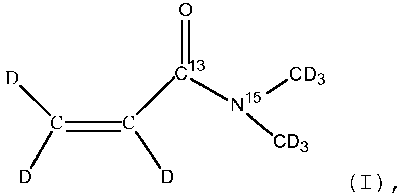 der hyperpolarisierten Verbindung der Formel II (2,2,3,3-Tetraflurorpropylacrylat ("TFPA")): 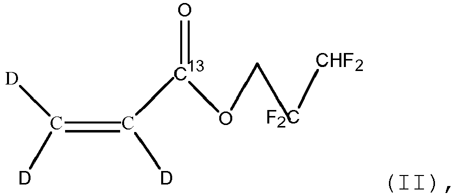 der hyperpolarisierten Verbindung der Formel III: 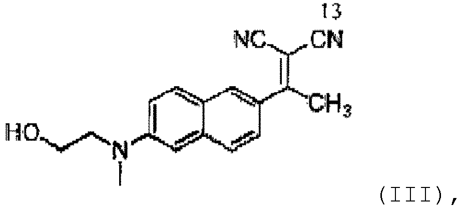 und Kombinationen davon.

18. Medizinische Vorrichtung nach Anspruch 17, wobei das Kontrastmittel wie in Anspruch 2 definiert ist.

19. Medizinische Vorrichtung nach Anspruch 17, wobei der Vorläufer oder das Substrat Diphenylacetylen ist, das Kontrastmittel ¹³C-Stilbamidin umfasst und die komplementäre Substanz aus der Gruppe bestehend aus einer Amyloid-Plaque, einer β-Amyloid-Plaque, Acetylcholinesterase und Kombinationen davon ausgewählt ist.

## Revendications

1. Procédé d'imagerie par résonance magnétique d'un sujet, comprenant :
l'exposition d'un sujet, auquel a été administré un agent de contraste préparé en faisant réagir de l'hydrogène enrichi en parahydrogène avec un précurseur ou un substrat d'un agent d'imagerie par résonance magnétique hydrogénable comprenant un noyau à spin nucléaire non-hydrogène, non-zéro, dans lequel l'agent de contraste est adapté pour cibler une substance complémentaire chez le sujet,
l'irradiation avec fréquence choisie pour exciter des transitions de spin nucléaire du noyau à spin nucléaire non-zéro dans l'agent de contraste ; et
la détection de signaux de résonance magnétique du noyau à spin nucléaire non-zéro provenant du sujet,
dans lequel le précurseur ou le substrat est choisi dans le groupe composé du succinate hyper-polarisé, du diphénylacétylène hyper-polarisé, du stilbène hyper-polarisé, du glucose hyper-polarisé, du déshydroglucose hyper-polarisé, du pyruvate de phosphoénol, du fumarate hyper-polarisé, du glutamate hyper-polarisé, 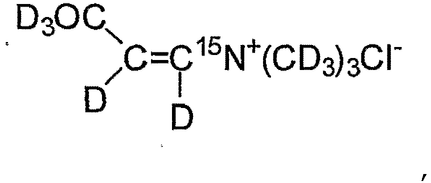 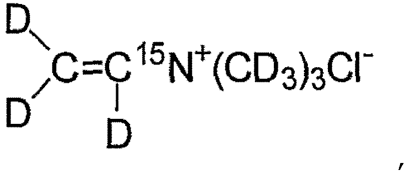 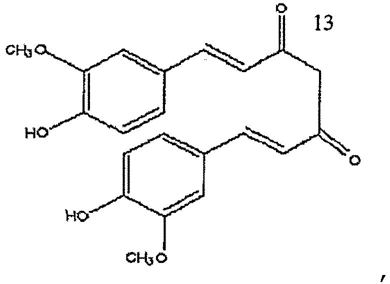 le composé hyper-polarisé de Formule I : 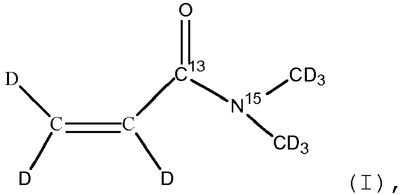 le composé hyper-polarisé de Formule II (acrylate de 2,2,3,3-tetraflroro-propyle (« TFPA »)) : 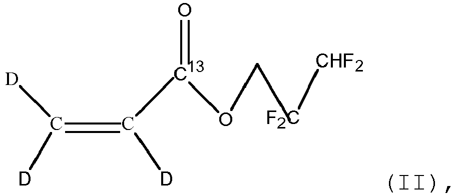 le composé hyper-polarisé de Formule III : 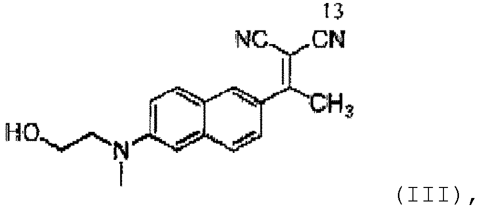 et des combinaisons de ceux-ci.

2. Procédé selon la revendication 1, dans lequel le noyau à spin nucléaire non-zéro est le ¹³C.

3. Procédé selon la revendication 1, dans lequel le sujet est un mammifère.

4. Procédé selon la revendication 1, dans lequel suivant l'administration l'agent de contraste interagit biochimiquement avec la substance complémentaire chez le sujet.

5. Procédé d'imagerie par résonance magnétique d'un sujet, comprenant :
l'exposition d'un sujet, auquel a été administré un agent de contraste préparé en faisant réagir de l'hydrogène enrichi en parahydrogène avec un précurseur ou un substrat d'un agent d'imagerie par résonance magnétique hydrogénable comprenant un noyau à spin nucléaire non-hydrogène, non-zéro, dans lequel l'agent de contraste est adapté pour cibler une substance complémentaire chez le sujet,
l'irradiation avec fréquence choisie pour exciter des transitions de spin nucléaire du noyau à spin nucléaire non-zéro dans l'agent de contraste ; et
la détection de signaux de résonance magnétique du noyau à spin nucléaire non-zéro provenant du sujet,
dans lequel l'agent de contraste est le ¹³C-stilbamidine et le précurseur ou le substrat est un précurseur hydrogénable comprenant le ¹³C.

6. Procédé selon la revendication 1, dans lequel la substance complémentaire est choisie dans le groupe composé d'une plaque d'amyloïde, d'une plaque d'amyloïde *β*, de l'acétylcholinestérase, et des combinaisons de celles-ci.

7. Procédé selon la revendication 1, dans lequel le précurseur ou le substrat est le déshydroglucose hyper-polarisé et la substance complémentaire est desneurones.

8. Procédé selon la revendication 1, dans lequel le précurseur ou le substrat est le pyruvate de phosphoénol et la substance complémentaire est des leucocytes.

9. Procédé selon la revendication 1, dans lequel le précurseur ou le substrat est le fumarate hyper-polarisé, le succinate hyper-polarisé, ou une combinaison de ceux-ci et la substance complémentaire est des neurones.

10. Procédé selon la revendication 1, dans lequel le précurseur ou le substrat est le glutamate hyper-polarisé et la substance complémentaire est des cellules immunitaires.

11. Procédé selon la revendication 1, dans lequel le précurseur ou le substrat est le composé hyper-polarisé de Formule I et la substance complémentaire est des cellules immunitaires.

12. Procédé selon la revendication 1, dans lequel le précurseur ou le substrat est le composé hyper-polarisé de Formule II et la substance complémentaire est une plaque d'athérosclérose et/ou des cellules immunitaires.

13. Trousse pour une imagerie par résonance magnétique, comprenant :
un agent de contraste préparé en faisant réagir de l'hydrogène enrichi en parahydrogène avec un précurseur ou un substrat d'un agent d'imagerie par résonance magnétique hydrogénable comprenant un noyau à spin nucléaire non-hydrogène, non-zéro, dans lequel l'agent de contraste est conçu pour cibler une substance complémentaire chez le sujet ; et
des instructions pour administrer l'agent de contraste chez le sujet, pour exposer le sujet au rayonnement avec une fréquence choisie pour exciter des transitions de spin nucléaire du noyau à spin nucléaire non-zéro dans l'agent de contraste, et pour détecter les signaux de résonance magnétique du noyau à spin nucléaire non-zéro provenant du sujet ;
dans lequel le précurseur ou le substrat est choisi dans le groupe composé du succinate hyper-polarisé, du diphénylacétylène hyper-polarisé, du stilbène hyper-polarisé, du glucose hyper-polarisé, du déshydroglucose hyper-polarisé, du pyruvate de phosphoénol, du fumarate hyper-polarisé, du 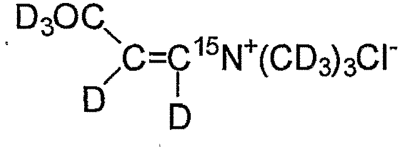 glutamate hyper-polarisé, 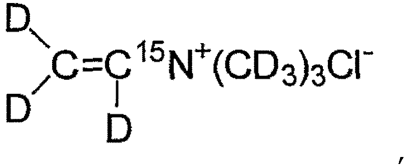 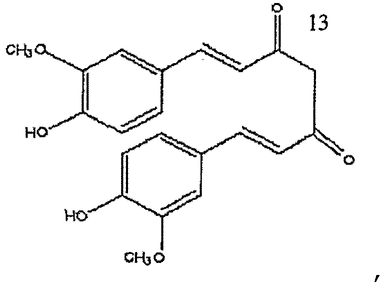 le composé hyper-polarisé de Formule I : 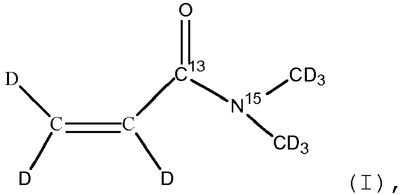 le composé hyper-polarisé de Formule II (acrylate de 2,2,3,3-tetraflroro-propyle (« TFPA »)) : 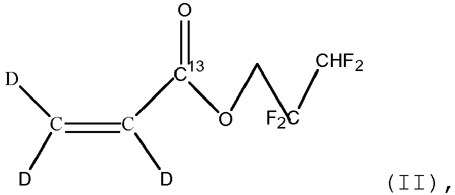 le composé hyper-polarisé de Formule III : 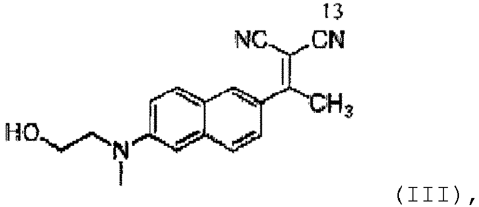 et des combinaisons de ceux-ci.

14. Trousse selon la revendication 13, dans laquelle les agents de contraste sont tels que définis dans l'une quelconque des revendications 1, 2 ou 4 à 6.

15. Trousse selon la revendication 13, dans laquelle le sujet est un mammifère.

16. Agent de contraste comprenant la ¹³C-stilbamidine pour une utilisation dans le diagnostic et/ou le pronostic de la maladie d'Alzheimer chez un mammifère,
dans lequel l'agent de contraste est conçu pour l'administration aux mammifères ;
dans lequel l'agent de contraste est préparé en faisant réagir de l'hydrogène enrichi en parahydrogène avec le diphénylacétylène ;
dans lequel l'agent de contraste est conçu pour cibler l'acétylcholinestérase, la plaque amyloïde et/ou la plaque amyloïde β chez le mammifère ;
dans lequel le mammifère est apte à être exposé au rayonnement à une fréquence choisie pour exciter les transitions de spin nucléaire d'un noyau à spin nucléaire non-zéro dans l'agent de contraste ;
dans lequel les signaux de résonance magnétique du noyau à spin nucléaire non-zéro sont détectés à partir du mammifère ; et
dans lequel la maladie d'Alzheimer chez le mammifère est diagnostiquée et/ou pronostiquée en se basant sur les signaux de résonance magnétique.

17. Dispositif médical pour une utilisation en relation à l'imagerie par résonance magnétique, comprenant :
un dispositif médical ; et
une quantité d'un agent de contraste imprégné sur ou enrobé sur le dispositif médical,
dans lequel l'agent de contraste est préparé en faisant réagir de l'hydrogène enrichi en parahydrogène avec un précurseur ou un substrat d'un agent d'imagerie par résonance magnétique hydrogénable comprenant un noyau à spin nucléaire non-hydrogène, non-zéro, et l'agent de contraste est conçu pour cibler une substance complémentaire chez le mammifère ;
dans lequel le précurseur ou le substrat est choisi dans le groupe composé du succinate hyper-polarisé, du diphénylacétylène hyper-polarisé, du stilbène hyper-polarisé, du glucose hyper-polarisé, du déshydroglucose hyper-polarisé, du pyruvate de phosphoénol, du fumarate hyper-polarisé, du 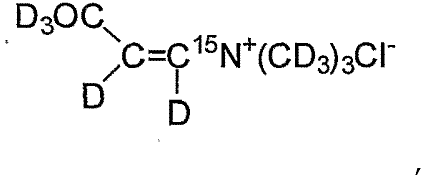 glutamate hyper-polarisé, 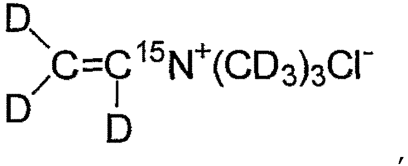 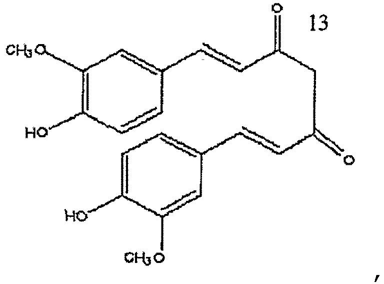 le composé hyper-polarisé de Formule I : 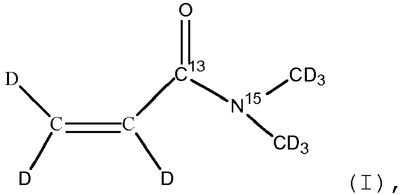 le composé hyper-polarisé de Formule II (acrylate de 2,2,3,3-tetraflroro-propyle (« TFPA »)) : 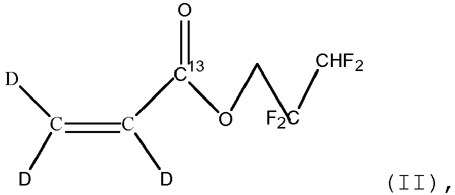 le composé hyper-polarisé de Formule III : 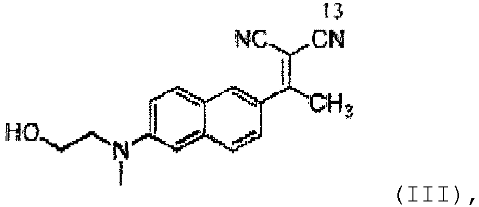 et des combinaisons de ceux-ci.

18. Dispositif médical selon la revendication 17, dans lequel l'agent de contraste est tel que défini dans la revendication 2.

19. Dispositif médical selon la revendication 17, dans lequel le précurseur ou le substrat est le diphénylacétylène, l'agent de contraste comprend la ¹³C-stilbamidine, et la substance complémentaire est choisie dans le groupe composé d'une plaque d'amyloïde, d'une plaque d'amyloïde *β*, de l'acétylcholinestérase, et des combinaisons de celles-ci.
